# EUROPEAN PATENT APPLICATION

(11) **EP 1 743 656 A2**
(43) Date of publication of application: **17.01.2007**
(21) Application number: 06122312.9
(22) Date of filing: 17.04.2003
(51) Int. Cl.: A61K 45/06, A61K 31/18, A61K 31/56, A61P 5/28, A61P 13/08

(54) **Pharmaceutical combination for the treatment of benign prostatic hyperplasia or for the long-term prevention of acute urinary retention**

(30) Priority: 24.04.2002 DE 10218392; 25.04.2002 DE 10218611
(62) Divisional of application: 03725053.7
(71) Applicant: Boehringer Ingelheim Pharma GmbH & Co. KG, 55216 Ingelheim am Rhein (DE)
(72) Inventor: The designation of the inventor has not yet been filed
(74) Representative: Hammann, Heinz

(57) **Abstract**

The present invention relates to a new pharmaceutical combination for treating benign prostatic hyperplasia (BPH) or for the long-term prevention of acute urinary retention (AUR).

## Description

### Field of the invention

The present invention relates to a pharmaceutical combination suitable for the treatment of benign prostatic hyperplasia or for the long-term prevention of acute urinary retention.

### Background to the invention

BPH, a benign, non-cancerous enlargement of the prostate, is a well-known complaint in men, which generally comes to medical attention from the age of 50 onwards. About 50% of all men aged over 50 and 95% of all men aged over 70 are affected by it. BPH is a generally progressive condition which in serious cases may endanger kidney function and require surgical intervention. The number of untreated patients runs at over 37 million worldwide. The reduced flow rate and voiding (obstructive) symptoms of BPH are generally considered to be caused by two main factors, the enlarged prostatic gland (the static component), and the tone of the smooth muscle of the stroma and urethra (the dynamic component). Storage (irritative) symptoms have been associated with bladder dysfunction secondary to outflow obstruction.

The growth and tone of the prostate compresses or lengthens the urethra, causing the symptoms of urethral blockage and possibly leading to urinary retention.

The prostate consists of epithelial glandular tubes embedded in fibromuscular stroma. Hyperplastic growth of the prostate begins at about the age of 30 in the periurethrally located parts of the gland, the so-called transition zone. Apart from the effects of ageing, androgenic hormones constitute a crucial stimulus to growth in the post-pubertal regulation of the volume of the gland. In the normal prostate, the enzyme 5α-reductase in the epithelial cells converts the androgenic hormone testosterone (T) into dihydrotestosterone (DHT). DHT, an active androgenic prostate metabolite, binds to cytoplasmic receptors and is transported into the cell nucleus where it initiates RNA and protein synthesis and cell replication. It is assumed that BPH occurs in response to the effects of DHT on the ageing prostate and to changes in the stroma and epithelial cells (Steers, Zorn, Dis. Mon., 41(7):437-497 (1995)).

Age-dependent changes in the serum concentrations of the hormonal regulatory circuit as a whole (LH, FSH, SHGB, T and DHT) and of other hormones which may affect this regulatory circuit (oestrogens, prolactin, testosterone derivatives), have been investigated as possible causes. However, there is no correlation between age-dependent hormonal changes in the serum and the intraprostatic hormone concentrations. Thus, it is clear that the prostate itself is responsible for regulating the hormonal milieu.

Possible points of attack for controlling the intraprostatic hormonal milieu are the 5α-reductase (i.e. the androgen metabolism), the hormone receptor expression in the epithelium and stroma, oestrogens and other hormones, and especially its type 2 isoform. In addition, numerous peptidal growth factors have a paracrine or autocrine effect on the local metabolism in the various compartments of the gland, by means of which the equilibrium of the cell kinetics can be shifted between proliferation and programmed cell death.

The clinical symptoms of BPH comprise both blocking (voiding, obstructive) symptoms (e.g. hesitancy to start urination, a weak or interrupted stream, post void urinary retention, acute urinary retention(AUR), stoppage of the stream of urine) which result directly from the constriction of the neck of the bladder and the prostatic urethra by the hyperplastic prostate, and also storage (irritative) symptoms of an irritated lower urinary tract (e.g. increased urinary frequency, nycturia, dysuria, urgency, urinary incontinence, uresiesthesis). Untreated, BPH may lead to serious complications of the urinary tract and kidneys such as e.g. acute urinary retention and hydronephrosis (uronephrosis).

Tamsulosin hydrochloride is an α-receptor blocker, a selective α₁-adrenoceptor antagonist, and is used as a monosubstance for treating functional symptoms of prostatic hyperplasia (BPH) or lower urinary tract symptoms (LUTS) associated with prostatic hyperplasia (BPH). Tamsulosin improves both storage and voiding symptoms in BPH patients by mediating the dynamic component of LUTS. The onset of action is fast and maintained on long-term treatment irrespective of prostate size. The need of BPH-surgery and catheterisation (mainly due to AUR) is significantly delayed.

The synthesis of tamsulosin and the acid addition salts thereof was first described in European Patent No. EP 34432, to which reference is hereby made.

A suitable sustained-release formulation is disclosed for example in US Patent No. 4,772,475, to which reference is also made.

5α-reductase inhibitors, and especially the inhibitors of the type 2 isoform of the enzyme, are also used to treat BPH. 5α-reductase inhibitors can reduce benign enlarged prostates (the static component of BPH) and consequently reduce storage and voiding symptoms to some extent. The onset of action is delayed but on long-term the risk for acute urinary retention and the need for BPH surgery can be reduced.

Examples of such inhibitors are the compounds described in US Patent No. 4,760,071, EP 285382, EP 285383 and WO 95/7927, and more specifically the compounds named finasteride (described for example in EP 155096 and US Patent No. 4,760,071) and dutasteride (described for example in WO 95/7927). Further compounds which have a 5α-reductase type 2 inhibitor activity may be determined using the assay described in Example 3 of WO 95/10284.

These compounds, as well as their pharmaceutically acceptable salts and their optically active isoforms, are all incorporated herein by reference.

### Summary of the invention

The present invention relates to a pharmaceutical combination containing tamsulosin and a testosterone 5α-reductase inhibitor. The combination according to the invention is suitable for the treatment of benign prostatic hyperplasia.

### Detailed description of the invention

Surprisingly, the combination according to the invention of tamsulosin, or an acid addition salt thereof, particularly tamsulosin hydrochloride, and a 5α-reductase inhibitor, particularly an inhibitor of the type 2 isoform of this enzyme, is suitable for therapy in the treatment of benign prostatic hyperplasia and shall offer additional benefit compared to each monotherapy.

In a preferred embodiment in accordance with the present invention, the 5α-reductase type 2 inhibitor is finasteride or dutasteride.

In a further preferred embodiment in accordance with the present invention, the combination is either a combination of tamsulosin and finasteride or a combination of tamsulosin and dutasteride, and this combination is especially suitable for long-term therapy in the treatment of benign prostatic hyperplasia.

In a further preferred embodiment, the combination according to the invention contains both active substances in a formulation which contains between 0.1 and 0.8 mg of tamsulosin hydrochloride and between 0.3 and 8 mg of the 5α-reductase type 2 inhibitor. The active substances may be administered orally.

In a further preferred embodiment, the combination according to the present invention is a solid pharmaceutical combination containing 0.1 to 0.8 mg of tamsulosin hydrochloride and 0.3 to 8 mg of the 5α-reductase type 2 inhibitor. The active substances may be administered orally.

In a further preferred embodiment, the combination according to the invention contains both active substances in a formulation which contains between 0.2 and 0.6 mg of tamsulosin hydrochloride and between 0.3 and 8 mg of the 5α-reductase type 2 inhibitor. The active substances may be administered orally.

In a further preferred embodiment, the combination according to the present invention is a pharmaceutical combination containing 0.2 to 0.6 mg of tamsulosin hydrochloride and 0.3 to 0.5 mg of the 5α-reductase type 2 inhibitor. The active substances may be administered orally and may be in a solid dosage form.

In a further preferred embodiment, the combination according to the present invention is a pharmaceutical combination containing 0.2 to 0.6 mg of tamsulosin hydrochloride and 0.3 to 8 mg of the 5α-reductase type 2 inhibitor. The active substances may be administered orally and may be in a solid dosage form.

Following examples of formulations are also intended to illustrate the invention.

A formulation which contains between 0.1 and 0.8 mg of tamsulosin hydrochloride and between 1 and 8 mg of finasteride.

A formulation which contains between 0.1 and 0.6 mg of tamsulosin hydrochloride and between 1 and 8 mg of finasteride.

A pharmaceutical combination containing 0.2 to 0.4 mg of tamsulosin hydrochloride and between 1 and 8 mg of finasteride.

A formulation which contains between 0.1 and 0.8 mg of tamsulosin hydrochloride and between 0.3 and 0.5 mg of finasteride.

A formulation which contains between 0.1 and 0.6 mg of tamsulosin hydrochloride and between 0.3 and 0.5 mg of finasteride.

A pharmaceutical combination containing 0.2 to 0.4 mg of tamsulosin hydrochloride and between 0.3 and 0.5 mg of finasteride.

A formulation which contains between 0.1 and 0.8 mg of tamsulosin hydrochloride and between 1 and 8 mg of dutasteride.

A formulation which contains between 0.1 and 0.6 mg of tamsulosin hydrochloride and between 1 and 8 mg of dutasteride.

A pharmaceutical combination containing 0.2 to 0.4 mg of tamsulosin hydrochloride and between 1 and 8 mg of dutasteride.

A formulation which contains between 0.1 and 0.8 mg of tamsulosin hydrochloride and between 0.3 and 0.5 mg of dutasteride.

A formulation which contains between 0.1 and 0.6 mg of tamsulosin hydrochloride and between 0.3 and 0.5 mg of dutasteride.

A pharmaceutical combination containing 0.2 to 0.4 mg of tamsulosin hydrochloride and between 0.3 and 0.5 mg of dutasteride.

These exemplified formulations may be in the form of a solid pharmaceutical dosage form and may be administered orally.

Suitable conventional preparations include, for example, inert conventional carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethyleneglycol, propyleneglycol, cetylstearyl alcohol, carboxymethylcellulose or fatty substances such as hard fat or suitable mixtures thereof, incorporated in conventional galenic preparations such as plain or coated tablets, capsules, powders, suspensions or suppositories.

The active substances may be given orally in a wide variety of different dosage forms: for example, they may be formulated together with various pharmaceutically acceptable inert carriers in the form of tablets, capsules, pastilles, lozenges, hard sweets, powders, aqueous suspensions, elixirs, syrups and the like. Carriers of this kind comprise, for example, solid diluents or fillers, sterile aqueous media and various non-toxic organic solvents. In addition, oral formulations of this kind may be suitably sweetened and/or flavoured with various agents normally used for this purpose. Generally, the active substances are present in oral dosage forms of this kind in concentrations ranging from about 0.5 % by weight to about 90 % by weight, based on the total composition, in amounts sufficient to produce the desired dosage units. Other dosage forms for the active substances include formulations for controlled release and devices which are well known to the specialists in the field.

Preferably, the two active substances are presented in a delayed-release formulation.

Within the meaning of the present invention, the two active substances may be present either in the same or in two separate formulations. In the latter case, the formulations containing the substances may be administered simultaneously or sequentially.

According to the invention, the expression long-term therapy denotes a medical application of the combination of two active substances in one or more formulations for at least 3 months or more.

The combination according to the invention appears to be especially suitable for treating patients with a particularly enlarged prostate and suffering from lower urinary tract symptoms (LUTS) and/or having a to be defined increased risk profile for disease progression, such as long-term prevention of acute urinary retention (AUR).

## Claims

1. Pharmaceutical combination containing the combination of tamsulosin, or an acid addition salt thereof, with a 5α-reductase inhibitor.

2. Pharmaceutical combination according to claim 1,
**characterised in that** the 5α-reductase inhibitor is an inhibitor of the type 2 isoform of the 5α-reductase enzyme.

3. Pharmaceutical combination in accordance with claim 1 or 2, **characterised in that** it contains tamsulosin hydrochloride.

4. Pharmaceutical combination in accordance with any one of claims 1 to 3, **characterised in that** the 5α-reductase inhibitor is finasteride.

5. Pharmaceutical combination in accordance with any one of claims 1 to 3, **characterised in that** the 5α-reductase inhibitor is dutasteride.

6. Pharmaceutical combination in accordance with any one of claims 1 to 5 for treating benign prostatic hyperplasia.

7. Pharmaceutical combination in accordance with any one of claims 1 to 6 for the long-term treatment of benign prostatic hyperplasia.

8. Pharmaceutical combination in accordance with any one of claims 1 to 5 for the long-term prevention of acute urinary retention.
